# EUROPÄISCHE PATENTSCHRIFT

(11) **EP 1 148 869 B1**
(45) Veröffentlichungstag und Bekanntmachung des Hinweises auf die Patenterteilung: **27.11.2002**
(21) Anmeldenummer: 99972529.4
(22) Anmeldetag: 27.10.1999
(51) Int. Cl.: A61K 9/00, B01D 69/02, B01D 67/00

(54) **VERFAHREN ZUM STEUERN EINES CHEMISCHEN VENTILES**
METHOD FOR REGULATING A CHEMICAL VALVE
PROCEDE POUR REGULER LA POROSITE D'UNE MEMBRANE

(30) Priorität: 19.11.1998 DE 19853286
(43) Veröffentlichungstag der Anmeldung: 31.10.2001
(73) Patentinhaber: Gesellschaft für Schwerionenforschung mbH, 64291 Darmstadt (DE)
(72) Erfinder: SPOHR, Reimar, D-64291 Darmstadt (DE); YOSHIDA, Masaru, Takasaki Gunma-Ken 370-12 (JP)
(86) Internationale Anmeldenummer: EP9908115
(87) Internationale Veröffentlichungsnummer: WO00030606

(56) Entgegenhaltungen:
- DE-A- 3 831 970
- US-A- 4 161 013
- US-A- 5 085 749
- US-A- 5 556 528

## Beschreibung

Die vorliegende Erfindung betrifft ein Verfahren zum Steuern eines chemischen Ventiles gemäß dem Oberbegriff des Patentanspruches 1.

Als chemische Ventile bezeichnet man poröse Membranen, deren Porositätseigenschaften durch im allgemeinsten Sinn chemische oder auch thermisch-physikalische Prozesse gesteuert werden können.

Ein chemisches Ventil mit einer porösen Matrix und einer durch ihre Umgebungsbedingungen quellbaren Gelschicht, bei dem die Matrix eine Mikroporenmembran ist, deren Poren durch Ätzen von Teilchenspuren in der Matrix erzeugt sind, ist aus der DE-PS 43 05 979 C2 bekannt.

Die Permeation von gelösten Stoffen durch solche stimulusresponsive Membranen lässt sich durch externe Reize, wie z.B. Temperatur, pH-Wert oder Konzentration regeln. Der Stofftransport wird durch das vorgegebene Konzentrations-bzw. Druckgefälle bestimmt. Dadurch bedingt, lässt sich der Stofftransport nicht elektrisch überwachen, nur langsam mit Zeitkonstanten im Minuten- und Stundenbereich regeln, nicht vollständig und kurzfristig ein- und ab-, sowie vollständig ausschalten.

Ein Verfahren, mit dem der Transport von Molekülen durch eine polyelektrolytische Membran dynamisch gesteuert wird, wird in der US 5,085,749 beschrieben. Chemische Modulationen von elektrostatisch schwellenden Kräften in der Membran zeigen sich im starken Wechsel in der Permeabilität und Selektivität. Ein transmembranes elektrisches Feld bewirkt elektroosmotische, elektrophoretische und elektrostatische Effekte, die kombiniert verwendet den selektiven Transport von Molekülen durch die Membran erlauben.

Ein elektromechanochemikalisches Gerät wird in der US 4,161,013 vorgestellt. Ein polyelektrolytischer Film, der aus molekularen Gruppen fester Ladung aufgebaut ist, ist in einen wässerigen Elektrolyt eingetaucht, der bewegliche Ionen enthält. Dieser Film wird einem elektrischen Feld ausgesetzt, das derart wirkt, daß das interne Profil der beweglichen Ionen innerhalb des polyelektrischen Films in solcher Art gesteuert wird, daß die Anordnung der Moleküle, die die Matrix des Films aufbauen, beeinflußt wird.

Strukturen mit feldresponsiver Steuerung der Permeation werden in der US 5,556,528 beschrieben. Geräte mit solchen Strukturen sind nützlich, Zielmoleküle einzufangen oder abzugeben. Sie haben eine elektrisches-Feld-responsives Ventil. Das Ventil hat eine aktive Steuerstruktur, die wenigstens eine monomolekulare Schicht bis zu 10 nm Dicke hat. Die aktive Steuerstruktur besteht aus einer Mehrheit von molekularen Spezies mit einem dipolaren Moment größer aals 5 Debye. Die Steuerstruktur ist durch die Antwort auf ein elektrisches Feld mit einem Grenzwert zu betreiben. Eine Ausführung der Steuerstruktur trägt eine mikroporöse Membrane im nm-Bereich. Anwendungen sind Medikamentenlieferung und Zielmoleküleinfang während der elektrophoretischen Trennung.

Davon ausgehend ist es Aufgabe der vorliegenden Erfindung, ein Verfahren anzugeben, mit welchem bei einem chemischem Ventil mit einer Membran der angegebenen Art obige Nachteile vermieden und die gewünschten positiven Eigenschaften erzielt werden können. Das Verfahren sollte eine eingebaute Sensorfunktion mit direkter elektrischer Überwachbarkeit des Stofftransportes ermöglichen, extrem schnell im Millisekundenbereich regelbar und damit u.a. für die Abgabe von Pharmaka geeignet sein. Dabei sollte die maximale Permeabilität chemisch vorgebbar und der Stofftransport kurzfristig ein- und abschaltbar sein.

Zur Lösung der Aufgabe schlägt die vorliegende Erfindung ein Verfahren mit den Merkmalen vor, die im kennzeichnenden Teil des Patentanspruches 1 angeführt sind. Ein weiterer vorteilhafter Verfahrensschritt ist in dem kennzeichnenden Teil des Unteranspruchs 2 mit dem ein- oder beidseitigen chemischen Reiz zu sehen.

Durch die erfindungsgemäße Kombination einer responsiven Membran mit einer extern schaltbaren Stromquelle ensteht ein chemisches Ventil mit einem Membransystem, das eine eingebaute Sensorfunktion mit direkter elektrischer Überwachbarkeit des Stofftransportes beinhaltet. Dies ermöglicht extrem schnelle Regelungsvorgänge, z.B. die Abgabe von Pharmaka, mit Zeitkonstanten im Milli- bis Sekundenbereich, wobei die maximale Permeabilität des chemischen Ventiles chemisch vorgebbar ist und sein Stofftransport sich vollständig und kurzfristig über das Ein- und Abschalten der Spannungsquelle und damit der Potentialdifferenz zwischen den beiden Elektrolyten steuern lässt.

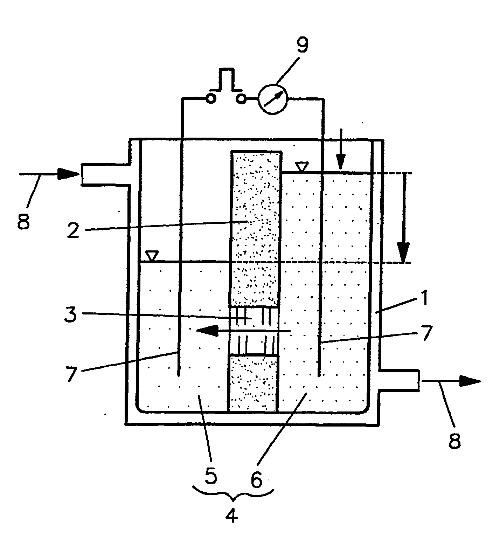

Einzelheiten des Verfahrens werden im folgenden und anhand der Figur, die das Prinzip eines auf dem Verfahren beruhenden neuen chemischen Ventiles mit einer Ionenspurmembran zeigt, näher erläutert:

In einer Zelle 1 ist eine thermo-oder chemisch reponsive nanoporöse Membran, wie z.B. eine mit einem Glucose-responsiven Gel ausgekleidete Mikroporenmembran 2 eingesetzt, die in vereinfachter Darstellung eine einzelne in dem responsiven Gel befindliche Pore 3 aufweist, wobei auch beliebig viele Poren in der Membran möglich sind. Die Pore/n weisen charakteristische Durchmesser im Bereich von 10 bis etwa 1000nm auf. Die durch einen Kreislauf 8 temperierte Zelle 1 ist mit einer wässrigen Lösung 4 als Elektrolyt gefüllt, wobei der wirksame Durchmesser der Poren 3 durch die Glucose-Konzentration in den beiden Elektrolytbereichen 5 und 6 der Lösung 4 als Grundwert eingestellt wird. In die beiden Elektrolytbereiche 5 und 6 auf beiden Seiten der Membran 2 sind je eine vorzugsweise nicht polarisierbare Elektrode 7 aus z.B. Ag/AgCl eingetaucht, an welche mittels der Stromquelle 9 eine elektrische Wechsel- oder Gleichspannung angelegt wird. Der Elektrolytbereich 5 ist dabei als der Innenraum eines Systemes, in welchem sich ein abzugebender Stoff befindet und der Elektrolytbereich 6 als der Außenraum bezeichnet, in den der durch die Pore/n 3 transportierte Stoff zu irgend einer Reaktion abgegeben wird. Die an die Elektroden 7 angelegte Wechsel- oder Gleichspannung führt nun zu einem elektrischen Strom durch die Pore/n 3, der je nach seiner Richtung einen elektro-osmotischen Druck vom Außenraum 6 zum Innenraum 5 oder umgekehrt und zusätzlich oder allein für sich gleichzeitig eine Temperaturveränderung in der/den Pore/n 3 bewirkt.

Die elektro-thermische Steuerung geschieht dabei wie folgt: Die thermo-responsive Membran 2 wird in die durch einen externen Thermostaten vortemperierte Zelle 1, z.B. eine Leitwertmeßzelle, eingebaut. Die Zelle 1 besitzt, wie bereits erwähnt, die beiden Elektrolytbereiche 5 und 6, die durch die Membran 2 voneinander getrennt sind. In der einen Hälfte, dem Innenraum 5 befindet sich der an die andere Hälfte, dem Außenraum 6 abzugebende Stoff, z.B. Insulin in wässriger Lösung unter einem hydrostatischen Druck zwischen 0 und 10 bar. Sobald nun über die Elektroden 7 ein elektrischer Gleich- oder Wechselstrom durch die Zelle 1 geschickt wird, erwärmt sich die Flüssigkeit in der/den Pore/n 3 der thermo-responsiven Membran 2. Dadurch wird diese rasch auf eine Temperatur oberhalb der kritischen Temperatur z.B. eine Hydrogels erwärmt, wobei die Membran 2 bzw. die Pore/n 3 schrumpfen und der abzugebende Stoff vom Innenraum 5 in den Außenraum 6 gepreßt wird. Sobald der elektrische Strom unterbrochen wird, kühlt sich die Membran 2 bzw. die Pore/n 3 wieder ab, das Hydrogel quillt und der Volumenstrom durch die Pore/n 3 wird unterbrochen. Die Stoffabgabe läßt sich dadurch im Sekundenbis Minutenbereich steuern.

Zur elektro-osmotischen Steuerung der Membran 2 wird bei teilweise geöffneter/n Pore/n 3 durch entsprechende Polung der Spannung der externen Stromquelle 9 mittels der Elektroden 7 über die Pore/n 3 bzw. von einem Elektrolytbereich 5 und 6 zum anderen durch die Spannungsdifferenz ein elektro-osmotischer Druck erzeugt, der dem hydrostatischen Druck unterschiedlicher Flüssigkeitsspiegel der beiden Elektrolytbereiche 5 und 6 entweder entgegen- oder gleichgerichtet ist. Bei dieser Methode ist eine gegenüber der elektro-thermischen Steuerung schneller ansprechende vollständige Unterdrückung bzw. erhöhte Stoffabgabe durch die Pore/n 3 möglich. Die Stoffabgabe läßt sich hierbei im Millisekunden- bis Sekundenbereich steuern.

Bei den genannten Verfahrensschritten kann die Mikroporenmembran zusätzlich ein- oder beidseitig einem chemischen Reiz ausgesetzt werden, wobei ein chemischer Reiz bei einer mit Glucose-responsivem Gel ausgekleideten Membran aus dem Konzentrationsgradienten von Glucose Molekülen in einer wässrigen Lösung als Elektrolyt bestehen kann..

Als Ausführungsbeispiel, Schaltung der Membran 2 durch Erwärmung des Hydrogels in einer Vielzahl von Poren 3 der Membran bzw. durch elektro-osmotisch erzeugte Drücke, wird im folgenden die Herstellung einer thermo -responsiven, elektrisch schaltbaren, nanoporösen Membran 2 beschrieben:

### 1. Herstellung einer Ionenspur-Filtermembran:

Ein 19µm dicker Polyethyleneterephtalate-Film wird mit (5,4±0,5)x10⁵ Gold Ionen von 11,6 MeV/Nukleon (MeV/u) pro cm² bestrahlt und in 5 N NaOH Lösung bei 40°C während 260 min geätzt. Dies führt zu ungefähr zylindrischen Poren mit Durchmessern von 2.9±0,2 µm und Längen von 17,7 µm.

### 2. Pfropfen der thermo-responsiven Gelschicht:

Zum chemischen Propfen wird die geätzte Ionenspurmembran in einer 10 gewichtsprozentigen ausgegasten wässrigen Lösung eines N-isopropylacrylamide Monomers getränkt und bei 60°C im Vakuum mit Gammastrahlen einer ⁶⁰Co-Quelle bei einer Dosisrate von kGy pro Stunde bestrahlt. Hieraus ergibt sich eine thermo-responsive Membran mit den erforderlichen Eigenschaften.

Das erfindungsgemäße Verfahren beinhaltet nun eine ganze Reihe von Vorteilen:
- eine elektrische Sensorfunktion, d.h. der Stofftransport ist elektrisch überwachbar
- das Verfahren ist in ein Medikamenten-Abgabesystem integrierbar
- der apparative Aufwand für das Verfahren ist gering
- es ist ein elektro-osmotisches Pumpen und Ein/Aus Schalten möglich
- die Membran bedarf nur weniger Herstellungsschritte und ist monolitisch herstellbar
- durch hohe Porendichte ist ein hoher Durchsatz möglich, wobei Poren unterschiedlichster, auch asymetrischer Geometrie zum Einsatz kommen können.

### Bezugzeichenliste:

- 1: Zelle
- 2: nanoporöse Membran
- 3: responsive Pore
- 4: Elektrolyt
- 5: Elektrolytbereich Innenraum
- 6: Elektrolytbereich Außenraum
- 7: Elektroden
- 8: Temperierung
- 9: Stromquelle

## Patentansprüche

1. Verfahren zum Steuern eines chemischen Ventils,
das aus einer thermo-responsiven, elektrisch schaltbaren, nanoporösen Membran besteht, die aus einer Ionenspurfiltermembran, und einer thermo-responsiven, quellbaren Gelschicht hergestellt ist, und
zwei thermostatisierte Elektrolyte in einem Bad voneinander trennt und elektrisch isoliert,
bestehend aus den Schritten:
über eine elektrische Wechsel- oder Gleichspannungsquelle, die an zwei in die beiden Elektrolyte ragende Elektroden angeschlossen ist, wird eine vorgebbare Spannungsdifferenz zwischen den beiden, durch die Membran voneinander getrennten Elektrolyten erzeugt, die die Öffnung der durch ätzen von schweren Ionen in der Ionenspurfiltermembran eingebrachten mindestens einen Pore elektrothermisch oder elektroosmotisch steuert,
wobei die Temperatur dort und damit die Porenöffnung in der mindestens einen Pore durch den elektrischen Widerstand über die Porenlänge durch die Wahl der Höhe der Spannungsdifferenz gezielt gesteuert wird.

2. Verfahren nach Anspruch 1,
**dadurch gekennzeichnet,**
**daß** die Membran zusätzlich ein- oder beidseitig einem chemischen Reiz ausgesetzt wird, der bei einer mit Glucose-responsivem Gel ausgekleideten Membran aus dem Konzentrationsgradienten von Glucose Molekülen in einer wässrigen Lösung als Elektrolyt besteht.

## Claims

1. Method of controlling a chemical valve, which comprises a thermo-responsive, electrically switchable, nanoporous diaphragm, which is produced from an ion trace filter diaphragm, and a thermo-responsive, swellable gel layer, and which valve separates two thermostatically controlled electrolytes in a bath from each other and electrically isolates them, said method comprising the following steps:
a prescribable voltage difference between the two electrolytes, which are separated from each other by the diaphragm, is produced via an electrical source of alternating voltage or direct voltage, which source communicates with two electrodes protruding into the two electrolytes;
and said voltage difference electrothermically or electroosmotically controls the opening of the at least one pore, which has been introduced by etching the ion trace filter diaphragm,
the temperature there, and hence the pore opening in the at least one pore, being appropriately controlled by the electrical resistance over the pore length by the selection of the level of the voltage difference.

2. Method according to claim 1, **characterised in that** the diaphragm is additionally subject to a chemical stimulus on one side or on both sides, which stimulus is formed from the concentration gradient of glucose molecules in an aqueous solution as the electrolyte when the diaphragm is lined with glucose-responsive gel.

## Revendications

1. Procédé de commande d'une soupape chimique, constituée par une membrane nanoporeuse, thermo-réactive, commandable électriquement, fabriquée à partir d'une membrane filtrante à traces d'ions et d'une couche de gel gonflable, thermo-réactive, et de deux électrolytes thermostatés séparés l'un de l'autre dans un bain et électriquement isolés, ce procédé comportant les étapes suivantes : au moyen d'une source de courant électrique alternatif ou continu, raccordée à deux électrodes plongées dans les deux électrolytes, on établit une différence de tension prédéterminée entre les électrolytes séparés par la membrane et on commande électrothermiquement ou électro-osmotiquement l'ouverture d'au moins un pore réalisé par attaque chimique dans la membrane filtrante à traces d'ions, le choix de la hauteur de la différence de tension permettant de commander la température et donc l'ouverture du pore, dans au moins un pore, en fonction de la résistance électrique qu'il présente sur sa longueur.

2. Procédé selon la revendication 1,
**caractérisé en ce que**
la membrane est soumise de plus, d'un ou des deux côtés, à l'action d'un excitant chimique qui, avec une membrane garnie d'un gel de glucose réactif est constitué par le gradient de concentration des molécules de glucose dans une solution aqueuse en tant qu'électrolyte.
